(19) Europäisches Patentamt
European Patent Office
Office européen des brevets

(11) Publication number: 0 219 225 B1

# (12) EUROPEAN PATENT SPECIFICATION

(45) Date of publication of the patent specification: 22.02.89

(51) Int. Cl.⁴: **C07D 307/42**, C07D 307/52

(21) Application number: 86306998.5

(22) Date of filing: 10.09.86

(54) **Process for the preparation of ranitidine or acid addition salts thereof, and intermediates for this preparation.**

(30) Priority: 27.09.85 DK 4384/85

(43) Date of publication of application: 22.04.87 Bulletin 87/17

(45) Publication of the grant of the patent: 22.02.89 Bulletin 89/8

(84) Designated Contracting States: AT CH DE GB LI NL SE

(56) References cited: US-A- 4 399 294

(73) Proprietor: A/S Gea Farmaceutisk Fabrik, Holger Danskesvej 89, DK-2000 Frederiksberg(DK)

(72) Inventor: Alhede, Borge, Rypelyngvej 8, DK-2670 Greve Strand(DK)
Inventor: Clausen, Finn Priess, Enevangen 77, DK-3450 Allerod(DE)

(74) Representative: Hartley, David et al, c/o Withers & Rogers 4 Dyer's Buildings Holborn, London, EC1N 2JT(GB)

EP 0 219 225 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a special process for the preparation of ranitidine or acid addition salts thereof. The invention also relates to an intermediate for preparing ranitidine, and a further intermediate for preparing the firstmentioned intermediate.

Ranitidine, the systematic name of which is N-[2-[[[5-[(dimethylamino)methyl]-2-furanyl]methyl]thio]-ethyl]-N'-methyl-2-nitro-1,1-ethenediamine and which has the formula (I)

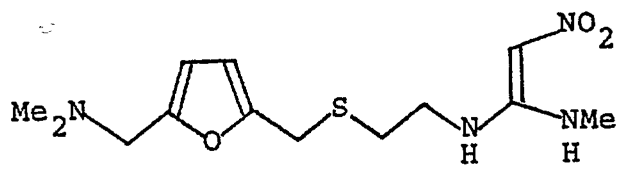

(I)

is a known, strongly acting selective histamine $H_2$ antagonist which especially is an important agent against gastric and duodenal ulcers. The original publication on the compound is GB patent specification No. 1 565 966 which also describes a number of processes for the preparation of ranitidine and related compounds. These are typical analogy processes which from readily available raw materials require a series of reaction steps and cumbersome purification methods, and give low yields.

Therefore several allegedly improved processes for the preparation of ranitidine have later appeared. The most important of these are to be discussed in the following.

Thus, US patent specification No. 4 497 961 describes and claims a process for the preparation of ranitidine, consisting in reacting a thiol having the formula (A)

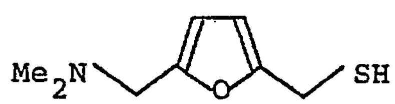

(A)

with an alkylating agent having the formula (B)

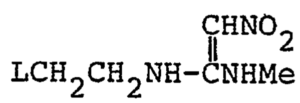

(B)

wherein L is a leaving group, preferably a halogen.

The yields, calculated on the thiol (A), seem to be of the order of magnitude 20–30%, and hence the overall yields, calculated on readily available furane derivatives, will be very low.

US patent specification No. 4 440 938 describes and claims the preparation of ranitidine by reacting a thiol (A) with an aziridine compound (ethyleneimine compound) having the formula (C)

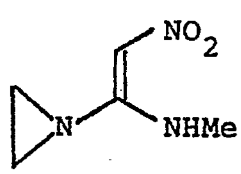

(C)

and GB patent specification No. 2 075 980 describes and claims the preparation of ranitidine by reacting (A) with (C); or related compounds by reacting similar compounds wherein the nitrogen atom in (A) and in (C), to which the Me groups are bonded, are otherwise substituted.

The said GB patent says that high yields are obtained in this reaction, viz. normally above 80% in this last reaction step for the preparation of ranitidine.

Even if the two starting materials are stated to be obtainable in fairly good yields, this process is not suited for use in technical scale because the aziridine (C) is empolyed. Aziridines are generally heavily toxic, very reactive substances which even in low concentrations act mutagenically and carcinogenically. The technical preparation and use of such compounds will be very precarious and necessitate costly security and control measures.

From European patent specification No. 55 626 B1 it is known for the preparation of ranitidine to react a compound having the formula (D)

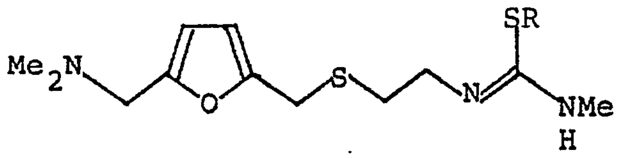

(D)

wherein R is $C_{1-6}$ alkyl at elevated temperature with nitromethane in large excess. The advantage is stated to be a reduced generation of thiol, but the examples of the specification show that the yields are low, at most 20%. Moreover, the use of nitromethane under these circumstances is not very suitable for industrial operation because of danger of explosion.

European patent specification No. 59 082 A1 describes the preparation of ranitidine by the reaction of 1-[[5-[(dimethylamino)methyl]-2-furanylmethyl]thio]-N-methyl-2-nitroethaneamine having the formula (E)

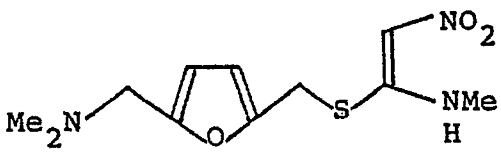

(E)

with aziridine. Although the yield according to the examples of the application is of the order of magnitude 35–45%, the process cannot be considered suitable for industrial utilization because of the use of the extremely poisonous and cancerogenic aziridine.

European patent specification No. 64 869 A1 describes the preparation of ranitidine from 5-[(dimethylamino)methyl]-2-furanemethanol and a disulphide having the formula (F)

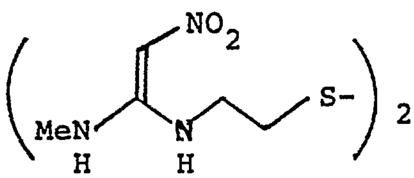

(F)

The advantage of this reaction is stated to be the use of inexpensive and readily available starting materials and the fact that the disulphide of the structure (F), which may be prepared from cystamine, can be easily isolated in pure crystalline form. This advantage, however, is totally eliminated by extremely low yields. Thus, in the last step of Example 1 of the application there is obtained a yield (based on the furan derivative) of about 3% solid material whereas Example 2 shows a yield of about 12% in the form of an oil.

Whereas in the processes discussed hereinabove there is employed a 2-substituted 5-[(dimethylamino)methyl]furan as starting material after which the part of the molecule to which the nitromethylene group is attached is completed, US patent specification No. 4 399 294 describes the preparation of ranitidine by treating an aldehyde of formula (G)

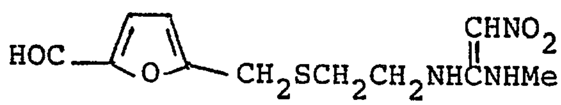

(G)

with dimethylamine and a reducing agent capable of effecting reductive alkylation to introduce the group $Me_2NCH_2$–. The aldehyde (G) is prepared from 2-[(aminoethyl)thiol]methylfurane having the formula (H)

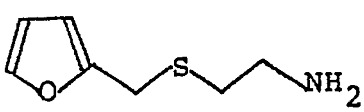

(H)

in a complicated reaction sequence.

It is not explained wherein the advantage of this process is believed to reside but at any rate it cannot be the size of the yield which, judging from the Examples is very low in the final step mentioned, and moreover the starting aldehyde is prepared in five steps from (H), wherein the amino group is protected by, e.g., a phthalimido group. The process thus must be considered as unsuitable for the preparation of ranitidine in commercial scale.

Accordingly, there is a strong need for an industrially usable process which from readily available raw materials provides ranitidine in technically simple reaction steps, in good yield and in high purity without using complicated purification methods.

This is obtained according to the invention, according to which ranitidine with the above formula (I) or an acid addition salt thereof is prepared by reacting N-[2-[[[5-(hydroxymethyl)-2-furanyl]methyl]thio]ethyl]-N'-methyl-2-nitro-1,1-ethenediamine having the formula (V)

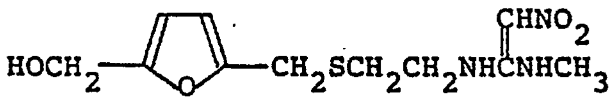

(V)

in an organic solvent, as for instance dimethylformamide, with dimethylamine and an N,N-(dimethylamino)triphenylphosphonium halide having the formula (VI)

$$(C_6H_5)_3\overset{+}{P}NMe_2,\ Hal^- \qquad (VI)$$

wherein Hal denotes bromo or chloro, after which the ranitidine thereby formed if desired is converted into an acid addition salt thereof.

The process according to the invention is illustrated in the reaction scheme below which also shows the preparation of the hitherto unknown compounds (IV) and (V) which constitute necessary intermediates.

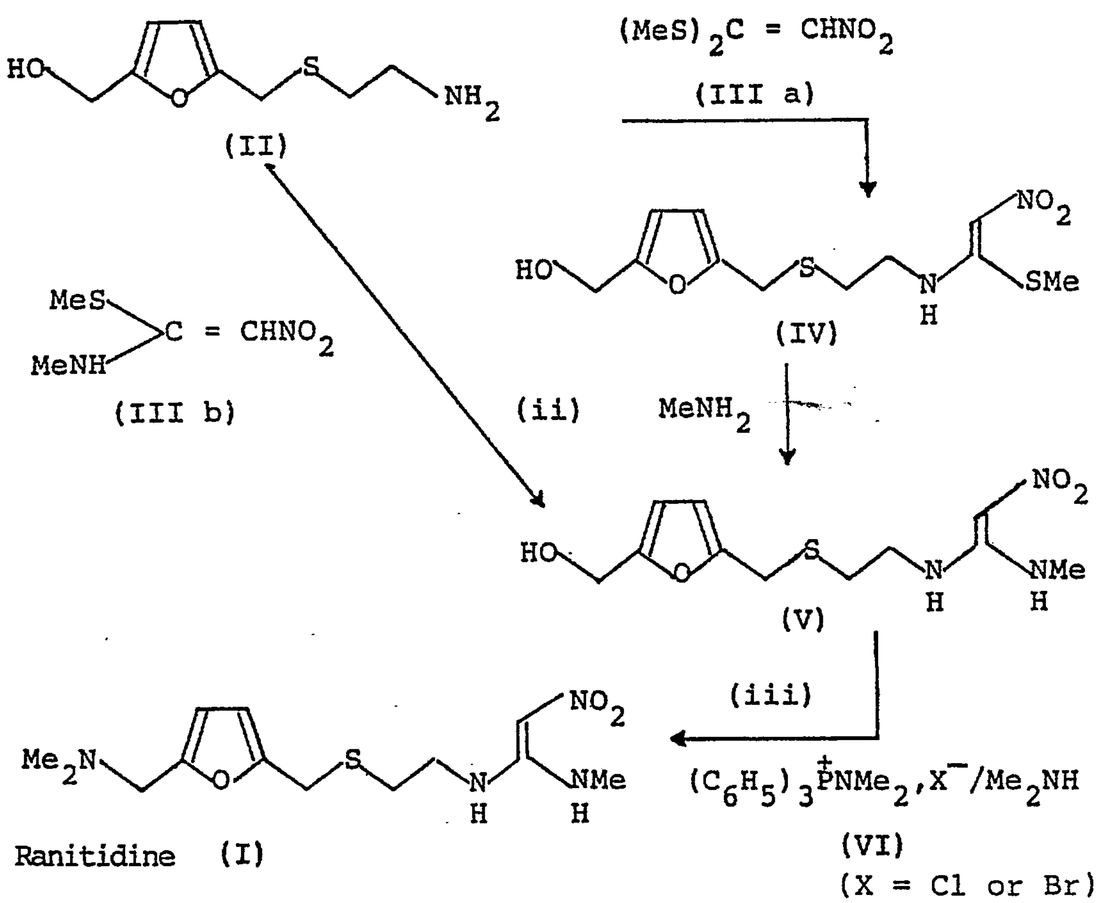


The starting compound 5-[[(2-aminoethyl)thio]methyl]-2-furanmethanol (II) is known from US patent specification No. 4 233 302, wherein the compound is called 2-[[5-(hydroxymethyl)-2-furanymethyl]thio]ethaneamine. The compound has not earlier been used for the preparation of ranitidine and there has not earlier been given physical data for it. According to Example 4 D in the said application it is prepared in four steps, in small yield (21%), from furfurylmercaptan. It has now been found that the compound can be prepared in another manner and in high yield (81%) from the commercially available 5-[(dimethylamino)methyl]-2-furanmethanol as shown in the following reaction scheme

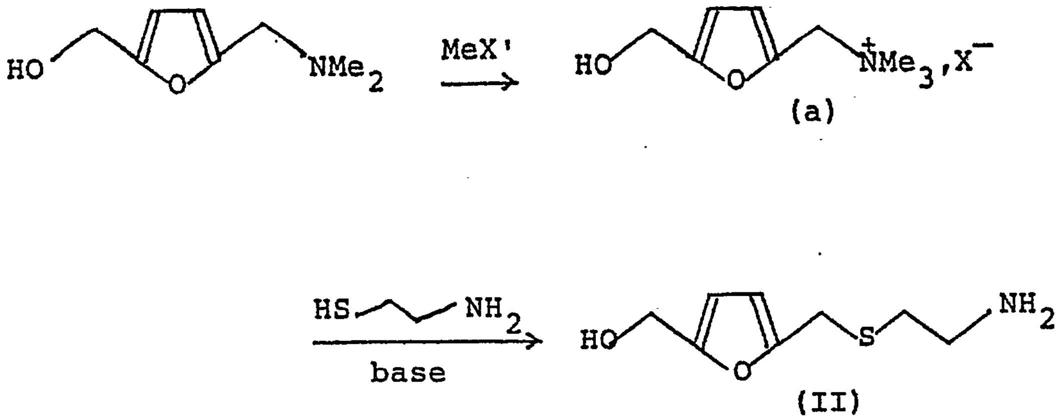


(a)

(II)

(X' = Cl, Br, I)

The reaction of compound (II) with the commercially available 1,1-bis(methylthio)-2-nitroethylene (IIIa) is carried out in organic solvents, preferably lower aliphatic alcohols or other lower-boiling polar solvents such as acetonitrile, whereby the compound (IV) can be obtained in a yield of 70–80%. The reaction is preferably carried out between room temperature and the boiling point of the solvent used.

The subsequent reaction of the compound (IV) with methylamine proceeds at moderate temperature, preferably between 0°C and 100°C in organic solvents as for instance lower alcohols and nitriles, whereby especially ethanol, methanol and acetonitrile have proven suitable. The compound (V) is obtained in a high yield (>90%) and the method is very suitable for industrial production.

It has surprisingly been found that the compound (V) by treatment with N,N-dimethylaminotriphenylphosphonium salts and dimethylamine in accordance with the invention, under relatively moderate conditions (about 90°C), in organic solvents (as for instance dimethylformamide) may be converted into ranitidine (I) in yields of 50–70%. In the process according to the invention ranitidine may be obtained as free base in high purity, or it may if desired be converted with an acid into a salt, e.g. the hydrochloride.

The reagent (VI) used in the reaction may be prepared in situ or in a stock solution by dissolving triphenylphosphine in an organic solvent as for instance dimethylformamide, thereafter adding chlorine or preferably bromine and finally dimethylamine in excess.

Like the preceding ones also step (III) is suitable for industrial production and the total reaction sequence does allow the preparation of ranitidine in an overall yield of about 50%, calculated on compound (II).

The described conversion of the intermediate (V) into ranitidine (I) in good yield under mild reaction conditions is very surprising since it is unknown to convert alcohols into dimethylamino compounds at moderate heating with dimethylaminotriphenyl phosphonium salts and dimethylamine.

It is also surprising that the intermediate in the reaction only reacts with the hydroxyl group and not, as could be expected, with the other side chain of the molecule, which other side chain contains a number of reactive groups.

A special advantage of the process according to the invention is that all starting materials are inexpensive, and in so far as they do not form part of the final product they may be easily regenerated (e.g. triphenylphosphine).

The invention also relates to the starting compound for the process according to the invention, i.e. N-[2-[[[5-(hydroxymethyl)-2-furanyl]methyl]thio]ethyl]-N'-methyl-2-nitro-1,1-ethenediamine of the formula (V), which is a novel compound:

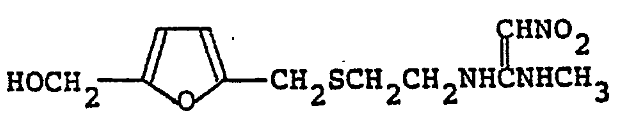

(V)

Finally, the invention relates to the novel compound (IV), which constitutes a starting material for the compound (V), and which is N-[2-[[[5-(hydroxymethyl)-2-furanyl]methyl]thio]ethyl]-1-methylthio-2-nitroetheneamine of the formula

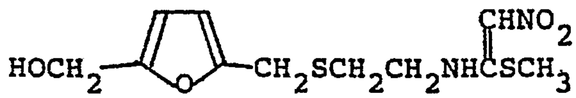

(IV)

The process according to the invention is to be illustrated more fully by the following Examples 8–10, whereas Examples 1–7 illustrate the preparation of starting materials and intermediates.

Example 1

5-(Hydroxymethyl)-N,N,N-trimethyl-2-furanmethaneaminium bromide (a)

Methyl bromide (38 g, 400 mmol) in acetone (100 ml) is added over 30 minutes to 5-[(dimethylamino)methyl]-2-furanmethanol (52.2 g, 336 mmol) dissolved in acetone (300 ml) at 20–30°C. After further 1 hour at 25°C the crystals formed are filtered off and washed with acetone. Drying at 60°C gives 81.4 g of the title compound (97%) (a) as white crystals. Mp. 151–153°C.
Calculated for $C_9H_{16}BrNO_2$: C 43.21 H 6.45 Br 31.95 N 5.60
Found: C 43.11 H 6.45 Br 32.00 N 5.46%

5-[[(2-Aminoethyl)thio]methyl]-2-furanmethanol (II)

5-(Hydroxymethyl)-N,N,N-trimethyl-2-furanemethane aminium bromide (a) (100.0 g, 400 mmol), cysteamine hydrochloride (48.0 g, 420 mmol) and 85% potassium hydroxide poweder (52.8 g, 800 mmol) are suspended in 1-butanol-toluene (1:1) (600 ml) and heated under nitrogen under reflux for 18 hours. After cooling at 25°C the suspension is washed with: 1) water (160 ml), 2) 25% sodium chloride solution (160 ml). The organic phase is evaporated in water jet vacuum after which the residue is dissolved in methanol (150 ml) and added to a solution of oxalic acid dihydrate (50.4 g, 400 mmol) in methanol (600 ml) at room temperature. The crystals formed are filtered off, washed with methanol and dried. Yield 91.5 g (83%) of (II), oxalic acid as an off-white crystalline product. Mp. 137–138°C (decomp.) with a purity of 95% (HPLC). By recrystallisation from methanol the mp. 140–141°C (decomp.) is obtained.
Calculated for $C_{10}H_{15}NO_6S$: C 43.31 H 5.45 N 5.05 S 11.57
Found: C 43.21 H 5.42 N 5.08 S 11.65%
Isolation of the title compound (II) as the free base:
Potassium hydroxide beads (30 g, 450 mmol) are added to the above oxalic acid salt of (II) (55.4 g, 200 mmol) in water (350 ml) and 1-butanol-toluene (1:1) (250 ml). The phases formed are separated and the aqueous phase is extracted with 1-butanol-toluene (1:1) (250 ml). The organic phases are combined and evaporated in water jet vacuum whereby there is obtained 37.4 g of the compound (II) as a pale lemon-coloured oil.

Example 2

N-[2-[[[5-(Hydroxymethyl)-2-furanyl]methyl]thio]ethyl]-1-methylthio-2-nitroetheneamine (IV)

Compound (II) (37.4 g, 200 mmol) dissolved in acetonitrile (60 ml) is added dropwise under reflux over 60 minutes to a solution of 1,1-bis(methylthio)-2-nitroethene (IIIa) (33.0 g, 200 mmol) in acetonitrile (300 ml). After reflux for further 4 hours 180 ml are distilled off. Stirring overnight at room temperature and thereafter 2 hours on an ice bath, filtration, wash with acetonitrile and drying gives 46.2 g (76%) of the title compound (IV) as a lemon-coloured powder. Mp. 97–100°C.
Calculated for $C_{11}H_{16}N_2O_4S_2$: C 43.40 H 5.30 N 9.21 S 21.07
Found: C 43.66 H 5.41 N 9.39 S 21.03%
The $^1H$ NMR spectrum of the product is in accordance with the structure assigned.

Example 3

N-[2-[[[5-(Hydroxymethyl)-2-furanyl]methyl]thio]ethyl]-1-methylthio-2-nitroetheneamine (IV) (from a, without purification of (II))

5-(Hydroxymethyl)-N,N,N-trimethyl-2-furanmethaneaminium bromide (a) (25.0 g, 100 mmol), cysteamine hydrochloride (12.0 g, 105 mmol) and 85% potassium hydroxide powder (13.2 g, 100 mmol) are suspended in 1-butanol-toluene (1:1) (150 ml) and heated under nitrogen at reflux for 18 hours. After cooling at 25°C the suspension is washed with: 1) water (40 ml), and 2) 25% sodium chloride solution (40 ml). The organic phase is evaporated in water jet vacuum to give 18.5 g of the crude compound (II). This is dissolved in acetonitrile (60 ml) and over 90 minutes dropwise added to 1,1-bis(methylthio)-2-nitroethene (IIIa) (16.5 g, 100 mmol) in acetonitrile (150 ml) under reflux. Continued reflux under nitrogen for 5 hours, stirring at room temperature overnight, cooling in an ice bath for 4 hours, filtration, wash with acetonitrile and drying gives 19.6 g of the title compound (IV) (65%, calculated on a) as a lemon-coloured powder with mp. 97–100°C.

Example 4

N-[2-[[[5-(Hydroxymethyl)-2-furanyl]methyl]thio]ethyl]-1-methylthio-2-nitroetheneamine (IV) (from a, without purification of (II))

Sodium (9.20 g, 400 mmol) is dissolved in ethanol (300 ml). There is cooled and cysteamine hydrochloride (22.8 g, 200 mmol) is added after which the mixture is stirred under nitrogen for 1 hour. 5-(Hydroxymethyl)-N,N,N-trimethyl-2-furanmethaneaminium bromide (a) (50.0 g, 200 mmol) is added. Thereafter there is refluxed under nitrogen for 20 hours. After evaporation to 150 ml precipitated salts are filtered off at 20°C. The filtrate is added over 2 hours under reflux to a solution of 1,1-bis-(methylthio)-2-nitroethene (IIIa) (32.2 g, 195 mmol) in ethanol (320 ml). After further 3 hours reflux the reaction mixture is stirred overnight at room temperature and thereupon at 0°C for 1 hour. Filtration, wash with ethanol and drying gives 27.7 g of the title compound (IV) (46%, calculated on a). Mp. 96–98°C.

Example 5

N-[2-[[[5-(Hydroxymethyl)-2-furanyl]methyl]thio]ethyl]-N'-methyl-2-nitro-1,1-ethenediamine (V)

N-[2-[[[5-Hydroxymethyl)-2-furanyl]methyl]thio]ethyl]-1-methylthio-2-nitroetheneamine (IV) (39.5 g, 130 mmol) is dissolved in 33%s methylamine in ethanol (190 ml) and stirred at room temperature for 20 hours. After evaporation to about 60 ml tetrahydrofuran (400 ml) is added. Stirring overnight at room temperature, filtration, wash with tetrahydrofuran and drying gives 34.6 g (93%) of the title compound (V) as a white powder. Mp. 105.5–108°C.
Calculated for $C_{11}H_{17}N_3O_4S$: C 45.98 H 5.97 N 14.62 S 11.16
Found: C 45.95 H 6.02 N 14.72 S 11.20%
$^1H$ NMR and $^{13}C$ NMR spectres of the product are in agreement with the structure assigned.

Example 6

N-[2-[[[5-(Hydroxymethyl)-2-furanyl]methyl]thio]ethyl]-N'-methyl-2-nitro-1,1-ethenediamine (V) (from (II), without purification of (IV))

5-[[(2-Aminoethyl)thio]methyl]-2-furanmethanol (II) (9.35 g, 50 mmol) in ethanol (30 ml) is added under nitrogen to a solution of 1,1-bis(methylthio)-2-nitroethene (IIIa) (8.25 g, 50 mmol) in ethanol (150 ml) under reflux over 45 minutes. After further reflux for 7 hours 33% methylamine in methanol (40 ml) is added whereafter the mixture is stirred overnight at room temperature. After evaporation tetrahydrofuran (100 ml) is added to the resulting oil. Stirring for 24 hours at room temperature, wash with tetrahydrofuran and drying give 9.80 g of the title compound (V) (68% calculated on (II)). Mp. 98–104°C. HPLC indicated the product to be 95% pure.

Example 7

N-[2-[[[5-(Hydroxymethyl)-2-furanyl]methyl]thio]ethyl]-N'-methyl-2-nitro-1,1-ethenediamine (V)

5-[[(2-Aminoethyl)thio]methyl]-2-furanmethanol (II) (2.8 g, 15 mmol) and 1-methylthio-1-methylamino-2-nitroethene (III b) (2.2 g, 15 mmol) are heated at reflux in ethanol (25 ml) under nitrogen for 4 hours. After evaporation to 10 ml and addition of tetrahydrofuran (35 ml) there is seeded and cooled at 0°C. Filtration, wash with tetrahydrofuran and drying give 1.7 g (37%) of the title compound (V) as a white product. Mp. 101–105°C.

Process according to the invention

Example 8

N-[2-[[[5-[(Dimethylamino)methyl]-2-furanyl]methyl]thio]ethyl]-N'-methyl-2-nitro-1,1-ethenediamine, hydrochloride (ranitidine hydrochloride), ((I), hydrochloride)

Bromine (13.9 g, 87 mmol) is added over 50 minutes while cooling to triphenylphosphine (23.6 g, 90 mmol) dissolved in dimethylformamide (65 ml). Dimethylamine (13.0 g, 290 mmol) is added over 30 minutes at 20–30°C under cooling to the resulting suspension. N-[2-[[[5-(Hydroxymethyl)-2-furanyl]methyl]thio]ethyl]-N'-methyl-2-nitro-1,1-ethenediamine (V) (8.61 g, 30 mmol) is added to this solution and the mixture is heated in a closed vessel at 90°C for 24 hours.

After cooling the reaction mixture is evaporated in water jet vacuum and 1-butanol/toluene (1:1) (100 ml), 12% sodium chloride solution (100 ml) and conc. hydrochloric acid (about 0.2 ml) are added to pH 3.5. The aqueous phase is thereafter washed with 1-butanol/toluene (1:1) (100 ml) and subsequently treated with active carbon at room temperature. To the filtered solution there is added 1-butanol/toluene (1:1) (100 ml) and there is adjusted at pH 9.0 with 11 N sodium hydroxide (about 2.6 ml). The aqueous phase is washed again with 1-butanol/toluene (1:1). The organic phases are washed with 12% sodium chloride solution (100 ml). The combined organic extracts are evaporated in water jet vacuum and dissolved in 2-propanol. The solution is filtered through a layer of silicagel (15 g). The filter layer is rinsed with 2-propanol. The filtrate is evaporated to 110 ml and 8 N hydrochloric acid (about 3.0 ml) is added to pH 4.0. The mixture is stirred overnight at room temperature and thereupon for 1 hour on an ice bath. The separated crystals are filtered off and washed with 2-propanol. Drying gives 7.02 g (67%) of ranitidine hydrochloride as a beige product. Mp. 135–139°C (decomp.).
Calculated for $C_{13}H_{23}ClN_4O_3S$: C 44.50 H 6.61 Cl 10.11 N 15.97 S 9.14
Found: C 44.26 H 6.67 Cl 10.26 N 16.00 S 9.13%

After recrystallization from dimethylformamide/ethyl acetate the product melted at 137–138°C (decomp.).

The $^1H$ NMR and IR spectres showed the product to be identical with authentic ranitidine hydrochloride.

Example 9

Ranitidine hydrochloride

The same method as in Example 8, but by using chlorine (6.2 g, 87 mmol) instead of bromine (13.9 g, 87 mmol). Yield: 48% ranitidine hydrochloride.

Example 10

Ranitidine hydrochloride

a) N,N-(Dimethylamino)-triphenylphosphonium bromide (VI)

Triphenylphosphine (78.6 g, 300 mmol) is dissolved in dimethylformamide (300 ml). Bromine (47.0 g, 294 mmol) is added over 40 minutes at 10–14°C. Dimethylamine (67 g, 1.5 mol) is introduced into the resulting suspension over 30 minutes at 20–30°C. The resulting solution of (VI) (0.63 mmol/g) is used directly in the next step.

b) N-[2-[[[5-[(Dimethylamino)-methyl]-2-furanyl]methyl]thio]-ethyl]-N'-methyl-2-nitro-1,1-ethenediamine hydrochloride (Ranitidine hydrochloride) ((I), hydrochloride)

N-[2-[[[5-(Hydroxymethyl)-2-furanyl]methyl]thio]ethyl]-N'-methyl-2-nitro-1,1-ethenediamine (V) (8.61 g, 30 mmol) is heated with the above solution of (VI) (98.0 g, 60 mmol) at 90°C in an autoclave for 20 hours. The reaction mixture is evaporated in water jet vacuum and 1-butanol-toluene (1:1) (100 ml), 12% sodium chloride solution (100 ml) and concentrated hydrochloric acid (0.5 ml) are added to pH 4.0. After stirring the phases formed are separated and the aqueous phase is washed with further portions of 1-butanol/toluene (1:1) (2 x 100 ml). 1-Butanol/toluene (1:1) (100 ml) is added to the aqueous phase and thereupon 9 N sodium hydroxide (4 ml) to pH 9.7. The aqueous phase is extracted with 1-butanol/toluene (1:1) (100 ml). The combined organic phases are evaporated in water jet vacuum and 2-propanol (80 ml) is added. This solution is filtered through a layer of silicagel (15 g). The filter layer is rinsed with 2-propanol. The filtrate is evaporated to 100 ml and 8 N hydrochloric acid (3 ml) is added to pH 4.0. After stirring overnight at room temperature there is filtered and washed with 2-propanol. Drying gives 6.1 g (58%) ranitidine hydrochloride as a beige product. HPLC indicated a purity of 96%. Mp. 130–134°C (decomp.).

## Claims

1. A process for the preparation of ranitidine, having the formula (I)

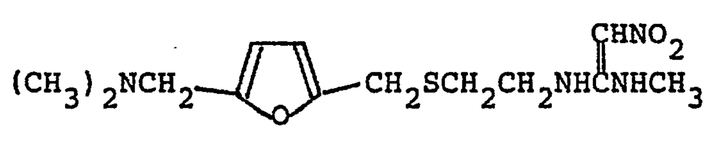

(I)

or an acid addition salt thereof, characterized in reacting N-[2-[[[5-(hydroxymethyl)-2-furanyl]methyl]thio]ethyl]-N'-methyl-2-nitro-1,1-ethenediamine having the formula (V)

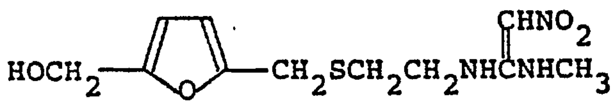 (V)

in an organic solvent with dimethylamine and an N,N-(dimethylamino)triphenylphosphonium halide having the formula (VI)

$$(C_6H_5)_3\overset{+}{P}NMe_2,\ Hal^-$$ (VI)

wherein Hal denotes bromo or chloro, after which the ranitidine thereby formed if desired is converted into an acid addition salt thereof.

2. A chemical compound, notably for use as starting material in the process defined in claim 1, characterized in that it is N-[2-[[[5-(hydroxymethyl)-2-furanyl]methyl]thio]ethyl]-N'-methyl-2-nitro-1,1-ethenediamine having the formula (V)

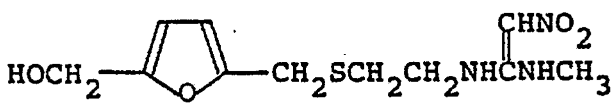 (V)

3. A chemical compound, notably for use as starting material for the preparation of the compound defined in claim 2, characterized in that it is N-[2-[[[5-(hydroxymethyl)-2-furanyl]methyl]thio]ethyl]-1-methylthio-2-nitroetheneamine having the formula (IV)

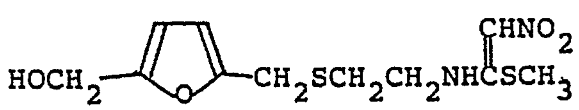 (IV)

**Patentansprüche**

1. Verfahren zur Herstellung von Ranitidin, das die Formel (I) hat

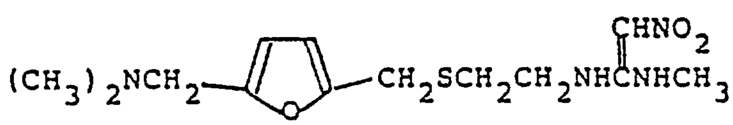 (I)

oder von einem Säureadditionssalz davon, dadurch gekennzeichnet, dass man N-[2-[[[5-(Hydroxymethyl)-2-furanyl]methyl]thio]ethyl]-N'-methyl-2-nitro-1,1-ethendiamin, das die Formel (V) hat

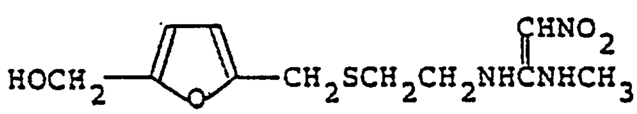 (V)

in einem organischen Lösungsmittel mit Dimethylamin und N,N-(Dimethylamino)triphenylphosphoniumhalogenid, das die Formel (VI) hat

$$(C_6H_5)_3\overset{+}{P}NMe_2,\ Hal^-$$ (VI)

worin Hal Brom oder Chlor bedeutet, umsetzt, wonach man das hierbei gebildete Ranitidin, falls erwünscht, in ein Säureadditionssalz davon umsetzt.

2. Chemische Verbindung, hervorragend zur Verwendung als Ausgangsmaterial im Verfahren, das in Anspruch 1 definiert ist, dadurch gekennzeichnet, dass sie N-[2-[[[5-(Hydroxymethyl)-2-furanyl]methyl]thio]ethyl]-N'-methyl-2-nitro-1,1-ethendiamin ist und die Formel (V) hat.

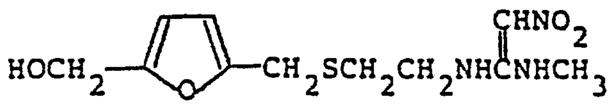 (V)

3. Chemische Verbindung, hervorragend zur Verwendung als Ausgangsmaterial für die Herstellung der Verbindung, die im Anspruch 2 definiert ist, dadurch gekennzeichnet, dass sie N-[2-[[[5-(Hydroxymethyl)-2-furanyl]methyl]thio]ethyl]-1-methyl-thio-2-nitroethenamin ist und die Formel (IV) hat

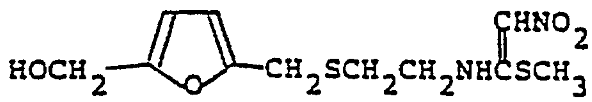 (IV) .

## Revendications

1. Procédé pour la préparation de ranitidine de la formule (I)

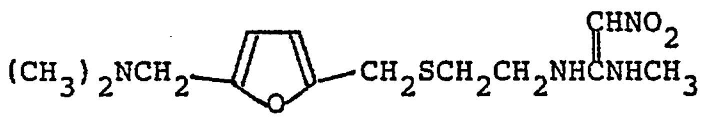 (I)

ou un sel d'addition d'acide de celle-ci, caractérisé en faisant réagir N-[2-[[[5-(hydroxyméthyl)-2-furanyl]méthyl]thio]éthyl]-N'-méthylnitro-2 éthènediamine-1,1 de la formule (V)

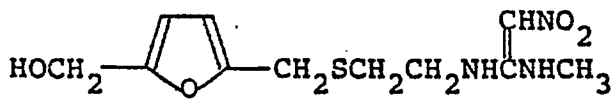 (V)

dans un solvant organique avec du diméthylamine et de l'halogénine N,N-diméthylaminotriphényle phosphonium de la formule (VI)

$$(C_6H_5)_3\overset{+}{P}NMe_2,\ Hal^- \qquad (VI)$$

où Hal représente chlore ou brome, la ranitidine étant ensuite au besoin convertie en un sel d'addition de ranitidine.

2. Composé chimique en particulier pour l'utilisation comme matériau de base dans le procédé selon la revendication 1, caractérisé en ce que ce composé est N-[2-[[[5-(hydroxyméthyl)-2-furanyl]méthyl]thio]éthyl]-N'-méthylnitro-2 éthènediamine-1,1 de formule (V).

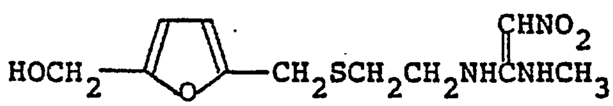 (V)

3. Composé chimique, en particulier pour l'utilisation comme matériau de base pour la préparation du composé selon la revendication 2, caractérisé en ce que c'est du N-[2-[[[5-(hydroxyméthyl)-2-furanyl]méthyl]thio]éthyl]-1-méthylthio nitro-2 éthèneamine de formule (IV).

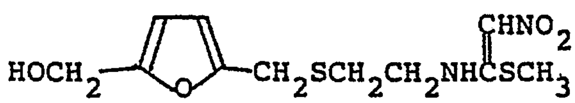 (IV)